(19) **Europäisches Patentamt European Patent Office Office européen des brevets**

(11) **EP 4 570 182 B1**

(12) **EUROPEAN PATENT SPECIFICATION**

(45) Date of publication and mention
of the grant of the patent:
**24.06.2026  Bulletin 2026/26**

(21) Application number: **23383275.7**

(22) Date of filing: **12.12.2023**

(51) International Patent Classification (IPC):
*A61B 6/58* (2024.01)          *A61B 6/50* (2024.01)

(52) Cooperative Patent Classification (CPC):
**A61B 6/582; A61B 6/505**

(54) **A COMPUTER-IMPLEMENTED METHOD, A SYSTEM, AND A COMPUTER PROGRAM FOR PHANTOMLESS CALIBRATION OF MEDICAL IMAGES**

COMPUTERIMPLEMENTIERTES VERFAHREN, SYSTEM UND COMPUTERPROGRAMM ZUR PHANTOMLOSEN KALIBRIERUNG MEDIZINISCHER BILDER

PROCÉDÉ MIS EN UVRE PAR ORDINATEUR, SYSTÈME ET PROGRAMME INFORMATIQUE POUR ÉTALONNAGE SANS FANTÔME D'IMAGES MÉDICALES

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**18.06.2025  Bulletin 2025/25**

(73) Proprietors:
• **3D-Shaper Medical S.L.**
  08007 Barcelona (ES)
• **Galgo Medical, S.L.**
  08007 Barcelona (ES)

(72) Inventors:
• **LÓPEZ PICAZO, Mirella**
  Barcelona (ES)
• **HUMBERT, Ludovic**
  Barcelona (ES)
• **THRISSUR RANGANATHAN, Sai Natarajan**
  Barcelona (ES)

(74) Representative: **Ponti & Partners, S.L.P**
**Edifici PRISMA**
**Av. Diagonal núm. 611-613, Planta 2**
**08028 Barcelona (ES)**

(56) References cited:
**US-A1- 2020 357 150**

• **JACKY C K CHOW ET AL: "Robust Self-Supervised Learning of Deterministic Errors in Single-Plane (Monoplanar) and Dual-Plane (Biplanar) X-ray Fluoroscopy", ARXIV.ORG, CORNELL UNIVERSITY LIBRARY, 201 OLIN LIBRARY CORNELL UNIVERSITY ITHACA, NY 14853, 3 January 2020 (2020-01-03), XP081571181, DOI: 10.1109/TMI.2019.2963446**

# EP 4 570 182 B1

**Description**

FIELD OF THE INVENTION

The present invention generally relates, in a first aspect, to a computer-implemented method for phantomless calibration of medical images, based on an artificial intelligence algorithm.

A second aspect of the present invention relates to a system for phantomless calibration of medical images adapted to implement the method of the first aspect.

A third aspect of the present invention relates to a computer program adapted to implement the method of the first aspect.

BACKGROUND OF THE INVENTION

Computed Tomography (CT) or Magnetic Resonance Imaging (MRI) are widely used imaging modalities that allow a non-invasive three-dimensional analysis of the internal organs and tissues. However, diagnosis of many pathological conditions requires a standardization of the image intensity data to allow a quantitative analysis of the affected organs or tissues. Quantitative assessment of body tissues and organs from CT scans (Quantitative Computed Tomography, QCT) is required in different areas of medicine, such as bone mineral density (BMD) assessment for osteoporosis diagnosis.

The absorption/attenuation coefficient of radiation within a tissue is used during CT reconstruction to produce a grayscale image. Grey-scale values in the CT scan (CT numbers), usually reported as a Hounsfield Unit (HU), are calculated based on a linear transformation of the baseline linear attenuation coefficient of the X-ray beam, where distilled water (at standard temperature and pressure) is arbitrarily defined to be zero HU and air defined as -1000 HU.

There are several factors in the process of acquiring a CT scan that affect the CT numbers, including the chemical composition of the structure being scanned, CT scanner characteristics, acquisition settings or reconstruction algorithm. Therefore, calibration of the CT numbers is required for quantitative assessment of BMD.

A possible solution to calibrate CT numbers is the placement of an external calibration phantom containing several different equivalent density tubes with known density values in the imaging field of view. The known density of the phantom tubes and the measured CT number in the phantom tubes are used to derive the linear relationship between CT number and equivalent density. A linear equation, particularly $density = a \times HU + b$, is calculated and applied to the CT image to convert CT numbers to bone density values.

Such reference inline phantoms have to be scanned together with the patient every time a CT acquisition is performed. Asynchronous phantom-based calibration uses a similar approach, except rather than scanning the calibration phantom simultaneously with the patient, the calibration phantom is scanned at a regular time interval (e.g., once a month), potentially with another phantom in place of the patient. The derived calibration is then applied to all patient scans acquired using the same acquisition parameters.

The main limitations of both inline and asynchronous phantom-based calibration are:

- Scanning the patient with an inline phantom is not a standard clinical practice.
- In many clinical centers, a calibration phantom (inline or asynchronous) is not available.
- The need to purchase a calibration phantom increases the economic cost of CT.
- The inline phantom complicates the acquisition protocol, because the phantom has to be scanned in every single CT acquisition.
- The phantom inserts need to be segmented to measure their apparent density in the image. Segmentation and measurement errors can alter the accuracy of the calculated calibration law.
- There are different phantoms with different inserts, and compositions, which result in different calibration laws.

To overcome these limitations, phantomless calibration methods were proposed to calibrate CT images without the need for a phantom. This enables the opportunistic assessment of CT images (including retrospective data) acquired without a phantom. Three approaches to phantomless calibration have been used in clinical research:

1. Direct use of CT numbers:
CT numbers are calibrated against water and can be used as estimates to measure bone density. An advantage of using such a simple approach is the avoidance of a calibration procedure. However, as CT numbers depend on CT scanners and acquisition parameters such as resolution, acquisition energy, or reconstruction kernel, this method provides an unreliable approach to measure bone density.
2. Using a generic law:
A generic law including subject-specific factors such as the age or the waist circumference could be used. One example of this approach can be found on patent KR101750108B1, which discloses the estimation of BMD using medical image signal without an external phantom, by using a generic calibration law with constant parameters (a and b) plus a subject-specific factors based on the waist circumference.

As a limitation, the generic law does not take into account differences in x-ray attenuation within the body due to the use of different CT scanners and/or acquisition parameters.

3. Internal calibration:

Internal calibration uses reference tissues with known densities, such as fat, muscle, blood, or air, within the CT image. The densities of these reference tissues are used to calculate a calibration equation, which is then applied to the CT image to convert HU to bone density values. This approach, however, comes with a few disadvantages:
Firstly, there is no standard method or agreement for determining which tissues to be used as references. Different approaches used different combinations of tissues and there is a debate in the literature on what tissues should be used to calculate accurate calibration laws (e.g. muscle and fat, muscle, fat, and air, etc.), as evidenced in [1, 2].
Secondly, the tissues of interest need to be segmented and their associated CT numbers measured in the CT images, which can be challenging for small structures such as blood vessels. The accuracy of the calibration equation can therefore be altered due to segmentation and measurement errors, as shown in [1, 3].
Finally, factors such as hydration levels, patient pathologies, heterogeneous distributions of muscle and fat, or intravenous contrast, influence the CT numbers of the reference soft tissues, and this could lead to errors in the calculation of the calibration laws.
US2020357150A1 discloses calibrating images having a truncated portion, based on a trained learning network, to obtain a predicted image of the truncated portion.
It is, therefore, necessary to provide an alternative to the state of the art by providing a computer-implemented method for phantomless calibration of medical images that overcomes the above-mentioned limitations.

SUMMARY OF THE INVENTION

To that end, the present invention relates, in a first aspect, to a computer-implemented method for phantomless calibration of medical images, comprising:

-    obtaining medical images without calibration phantoms of a plurality of previous subjects;
-    obtaining calibration laws for said obtained medical images without calibration phantoms;
-    training an artificial intelligence (AI) algorithm with at least one dataset of said obtained medical images without calibration phantoms and with said calibration laws (each with its respective calibration law), to learn a mathematical estimator that infers from image data the calibration coefficients of the calibration laws; and
-    calibrating with said at least one learned mathematical estimator at least one medical image of a current subject acquired without using a calibration phantom.

In the present invention, the "previous subjects" refer to those subjects from which image data (medical images) is obtained and used to develop the AI algorithm, while the "current subject" refer to the subject benefiting from the already trained AI algorithm, i.e. that subject who is scanned without using a calibration phantom and the obtained medical image is phantomless calibrated.
The image data contained in that current subject medical image is the one referred above from which the learned mathematical estimator infers the calibration coefficients of the calibration laws.
For a preferred embodiment, the method of the first aspect of the invention further comprises:

-    receiving and/or accessing medical images of said plurality of previous subjects scanned together with calibration phantoms; and
-    computing said calibration laws from said received and/or accessed medical images.

For an alternative embodiment, the calibration laws are obtained by scanning one or more calibration phantoms, such as those used in asynchronous calibration methods, where calibration data is obtained using intermittent calibration phantom scans, independently from the subject scans. Any calibration phantom suitable for this purpose could be used, whether a single-component phantom or a multi-component phantom.
For an embodiment of the method of the first aspect of the invention the step of obtaining medical images without calibration phantoms comprises generating medical images without calibration phantoms by removing, cropping or hiding image portions representing phantoms from medical images of the above-mentioned plurality of previous subjects scanned together with calibration phantoms.

For another embodiment, alternative or complementary to the above embodiment, the step of obtaining medical images without calibration phantoms comprises receiving and/or accessing medical images of the plurality of previous subjects scanned without calibration phantoms.

According to an embodiment, the medical images of the plurality of previous subjects scanned with calibration phantoms and the medical images of the plurality of previous subjects obtained without calibration phantoms are from the same body part, such as a femur.

For another embodiment, the medical images of the plurality of previous subjects scanned with calibration phantoms and the medical images of the plurality of previous subjects obtained without calibration phantoms include the same body part of a plurality of previous subjects. For example, the medical images of the plurality of previous subjects scanned with calibration phantoms could be of legs and the medical images of the plurality of previous subjects obtained without calibration phantoms of parts of those legs.

The method of the first aspect of the invention further comprises, for some embodiments, performing quantitative measurements, on one or more regions of interest (ROIs), of at least one of the following parameters of interest on said at least one calibrated medical image of a current subject acquired without using a calibration phantom: bone mineral density, mineral content in blood vessels, hepatic-fat content in the liver, and grey-to-white matter ratio in the brain, or a combination thereof.

Thus, depending on the embodiment of the method of the first aspect of the invention, different ROIs and parameters can be measured on the calibrated medical image(s), and the calibrated medical image(s) can be used for one or multiple purposes.

For an embodiment, the calibration laws are linear equations.

For some embodiments, the medical images of the plurality of previous subjects scanned with calibration phantoms and the at least one medical image of a current subject acquired without using a calibration phantom are computed tomography images and/or magnetic resonance images.

Depending on the embodiment, the medical images of the plurality of previous subject and/or that of the current subject are part or an entire 3D volume, a single slice (2D) of a 3D volume, a single slice in each plane stacked in a 3-channel format (2D+), selected slices stacked in a sequential manner from any one plane (2.5D), or a combination thereof. Other types of medical images are also embraced by the present invention.

For an implementation of the above embodiment for which medical images of the plurality of previous subjects scanned without calibration phantoms are received and/or accessed, those medical images are computed tomography images and/or magnetic resonance images.

According to an embodiment, the above mentioned at least one dataset comprises a training dataset and a validation dataset, the training step comprising inputting the same with the training dataset and the validation dataset and with said calibration laws, and wherein the method further comprises testing said trained artificial intelligence algorithm by inputting the same with a test dataset of said obtained medical images without calibration phantoms of the plurality of previous subjects (although, alternatively, a test dataset of medical images without calibration phantoms of current subjects could also be used to test the trained AI algorithm).

For an embodiment, the method of the first aspect of the invention comprises using the artificial intelligence algorithm to obtain the mathematical estimator by processing the whole area or volume of the obtained medical images without calibration phantoms, or of preprocessed images (filtered, smoothed, etc.) obtained therefrom, without segmenting or selecting specific image areas.

According to an embodiment, the obtained medical images without calibration phantoms of a plurality of previous subjects and the at least one medical image of a current subject acquired without using a calibration phantom represent or include a representation of the same body part.

For another embodiment, the obtained medical images without calibration phantoms of a plurality of previous subjects represent or include a representation of a body part, and the at least one medical image of the current subject acquired without using a calibration phantom represents or includes a representation of a different body part, the mathematical estimator being used to calibrate that at least one medical image representing or including a representation of that different body part.

Preferably, the received and/or accessed medical images of the plurality of previous subjects scanned without a calibration phantom were scanned with the same scanning system as the received and/or accessed medical images of the plurality of previous subjects scanned together with a calibration phantom. However, for other embodiments, different scanning systems can be used if they are sufficiently similar in operation and follow the same or similar operating protocol.

According to an embodiment, the calibration coefficients are learned by the artificial intelligence algorithm using multi-output regression.

For an embodiment, the artificial intelligence algorithm is a machine learning algorithm.

For an implementation of that embodiment, the machine learning algorithm is a deep learning algorithm.

For a variant of that embodiment, the deep learning algorithm implements a convolutional neural network, such as a squeeze-excitation residual network.

For another variant of that embodiment, the deep learning algorithm implements a neural network of compact convolutional transformers.

The method of the first aspect of the present invention constitutes a preferably fully automatic method to calibrate medical images without the use of an external calibration phantom or any predefined reference of tissue density to calibrate the image. The calibrated images could be used for quantitative assessment of internal body tissues (e.g., bone mineral density measurements) and organs and/or standardization of the image intensity data.

The present disclosure also includes further method steps for scanning the plurality of previous subjects together with calibration phantoms to obtain the above mentioned medical images, and for scanning the current subject without using a calibration phantom to obtain the above mentioned at least one medical image.

A second aspect of the present invention relates to a system for phantomless calibration of medical images, comprising a processing device including a processor and a computer-readable medium having encoded thereon computer-executable instructions to cause the processor to execute the computer-implemented method of the first aspect of the invention.

The present disclosure also includes a scanning system including and/or operatively connected to the system of the second aspect of the present invention to implement the above mentioned further method steps and the method of the first aspect of the present invention.

A third aspect of the present invention relates to a computer program, including code instructions that when executed on at least one processor perform the steps of the method of the first aspect of the invention, for phantomless calibration of medical images.

A computer program product comprising a tangible medium and, stored therein, the computer program of the third aspect of the present invention, is also provided by the present invention.

BRIEF DESCRIPTION OF THE FIGURES

In the following some preferred embodiments of the invention will be described with reference to the enclosed figures. They are provided only for illustration purposes without however limiting the scope of the invention. In accordance with common practice, the components in the figures are drawn to emphasize specific features and they are not drawn to the right scale.

Figure 1. A: a CT image with a calibration phantom obtained from the CT scan of a subject, for a phantom-based calibration. B: A plot representing a linear regression computed from that CT image, according to an embodiment of the method of the first aspect of the present invention.

Figure 2 shows one original CT image with phantom (left view) and the simulated CT image obtained once the phantom has been hidden (right view), according to an embodiment of the method of the first aspect of the present invention.

Figure 3 schematically shows the complete pipeline, for implementing part of an embodiment of the method of the first aspect of the present invention.

Figure 4 schematically shows the architecture of the AI model/algorithm used for some embodiments of the method of the first aspect of the invention.

Figure 5 schematically shows a linear regression module implementing part of the method of the first aspect of the invention, for an embodiment.

Figure 6 is a plot showing the train and validation curves for the AI algorithm of the method of the first aspect of the invention, for an embodiment.

Figure 7, left view, shows the performance of the method of the first aspect of the present invention on the prediction of slope estimates and, Figure 7, right view, shows the performance on the intercept estimates, when given a CT volume as input for the test dataset, for an embodiment.

Figure 8 shows two plots representing, left, how the integral vBMD measured using the CT images calibrated with the generic law correlates with the integral vBMD measured using the phantom-based calibrated CT images, and, right, the higher correlation found between the integral vBMD measured using the CT images calibrated with the method of the first aspect of the present invention, for an embodiment, and the integral vBMD measured using the phantom-based calibrated CT images.

Figure 9 shows two plots representing, left, how the trabecular vBMD measured using the CT images calibrated with the generic law correlates with the trabecular vBMD measured using the phantom-based calibrated CT images, and, right, the higher correlation found between the trabecular vBMD measured using the CT images calibrated with the method of the first aspect of the present invention, for an embodiment, and the trabecular vBMD measured using the phantom-based calibrated CT images.

Figure 10 shows two plots representing, left, how the cortical vBMD measured using the CT images calibrated with the generic law correlates with the cortical vBMD measured using the phantom-based calibrated CT images, and, right, the higher correlation found between the cortical vBMD measured using the CT images calibrated with the method of the first aspect of the present invention, for an embodiment, and the cortical vBMD measured using the phantom-based calibrated CT images..

DESCRIPTION OF THE PREFERRED EMBODIMENTS

An experimental embodiment was developed by the present inventors, where the method of the first aspect of the present invention was applied to the calibration of Computed Tomography (CT) images of the hip to measure bone mineral density (BMD) at the proximal femur, and is described below.

*Study population:*

A total of 597 hip CT scans were included in this study. The distribution of participants by gender was 84 male participants, 330 female participants, and 183 with unknown gender. The age range of participants was 19 to 92 years old. Participants had a large range of Bone Mineral Density (BMD), from normal bone density to osteoporosis. Exclusion criteria for all study subjects included skeletal disease other than osteoporosis and/or osteoarthritis, such as Paget's disease or cancer, and previous hip fracture.

*CT image acquisition:*

Study subjects were scanned using standard CT scanners together with a calibration phantom. A total of six different CT scanners were used, from two manufacturers (GE and Philips). The pixel size of the CT images ranged from 0.68 mm x 0.68 mm to 1.13 mm x 1.13 mm, with a distance between consecutive slices ranging from -0.50 mm to 1.00 mm and a slice thickness ranging from 1 mm to 2.5 mm. A convolution kernel of A was used for Philips scanners and a standard kernel was used for GE scanners. The X-ray source potential was 120 kVp and the tube current ranged from 138 to 188 mA, resulting in a dose index ranging from 10.0 to 11.6 mGy. The region of interest (ROI) was set to include both proximal femurs, with the most proximal slice positioned above the femoral head and the most distal slice below the lesser trochanter.

*Phantom-based calibration:*

The CT images were calibrated using the calibration phantom. The Hounsfield Units at each voxel were converted to bone density values according to the protocol recommended by the phantom manufacturer.

For each CT scan:

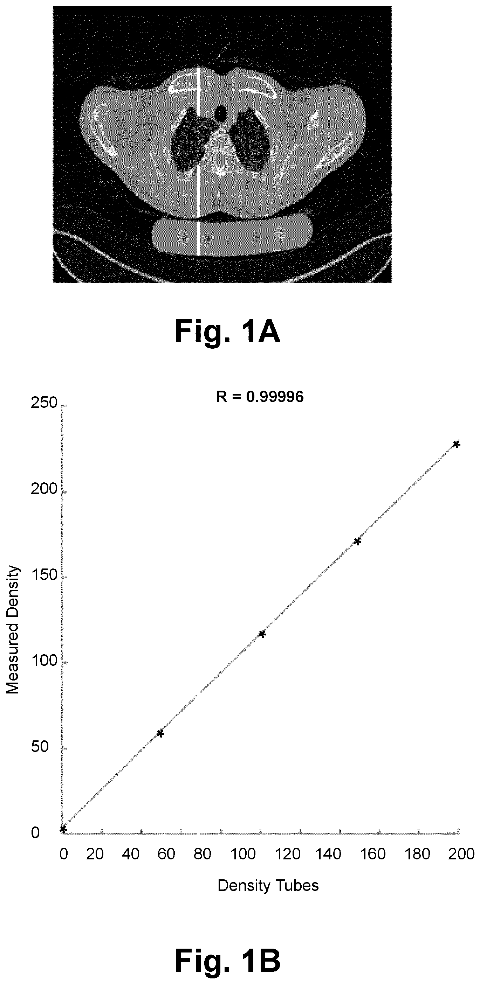

1. Identify the phantom tubes, Figure 1 left view. A point is manually placed in each phantom tube.
2. Compute a cylinder inside the phantom tube. A method has been developed to compute the cylinders automatically from the points selected.
3. Compute linear equation **Density** = a x **HU** + b using the theorical density of the tubes **(Density)** and the measured density **(HU)** of the cylinders (Figure 1 right view, where "Density of the tubes" refers to the density of the calibration tubes included in the calibration phantom, and "Measured density" to the bone density measured). Those phantom-based calibration equations will be used as ground truth calibration equations.

*AI-based phantomless calibration method, i.e. the method of the present invention:*

*- Overview of the methods and validation:*

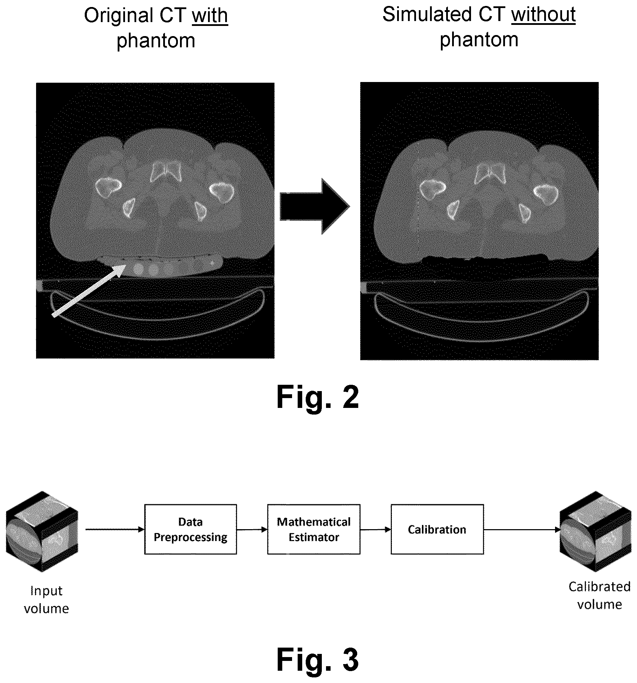

To simulate real-world CT volumes without calibration phantom, the present inventors developed a preprocessing method to hide the phantom in the acquired CT volumes. Figure 2 shows one of the original CT images with phantom (left view) of the acquired CT volumes and the simulated CT image obtained once the phantom has been hidden (right view).

The CT dataset was split into training, validation and testing. A Convolutional Neural Network (CNN) was trained using the training dataset of simulated CT images without calibration phantom, and their respective calibration laws (derived using the phantom-based calibration). The CNN algorithm extracts the proper features/tissues from the images to learn a mathematical estimator that infers the calibration coefficients of the calibration laws.

The validation dataset of simulated CT images without calibration phantom, and their respective calibration laws were used to validate and tune the CNN model.

The test dataset of simulated CT images without calibration phantom, was used to evaluate the performance of the mathematical estimator against the phantom-based calibration laws.

This method is completely automatic, and no manual intervention is needed once the model is trained.

Figure 3 schematically shows the complete pipeline, for implementing part of an embodiment of the method of the first aspect of the present invention, particularly once the AI algorithm, such as the one implementing the above mentioned CNN model, is already trained. The following stages are included in Figure 3, from left to right:

- an input CT volume, representing the above mentioned medical images of a current subject acquired without using a calibration phantom;
- a data preprocessing stage, for, for example, cropping, filtering or smoothing the inputted medical images;
- a mathematical estimator, implementing the AI network, where the preprocessed medical images are inputted and the slope and intercept parameters of the calibration laws, i.e. the above mentioned calibration coefficients, such as a and b in density = a × HU + b, are predicted.
- a calibration stage, where the inputted CT volume is calibrated with the learned mathematical estimator; and
- the obtained calibrated CT volume.

### Network Architecture:

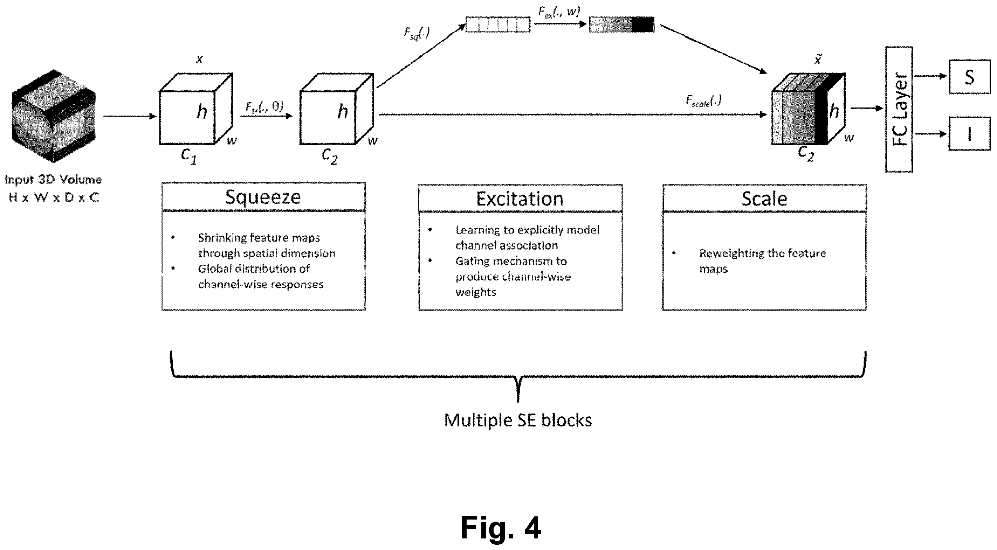

Figure 4 schematically shows the architecture of the AI model/algorithm used for the presently described embodiments, where the AI algorithm is a deep learning algorithm that implements a convolutional neural network, particularly a squeeze-excitation residual network.

### Notations:

In Figure 4, the following notations are included:

- **Input** 3D **Volume:** of size height (H) * width (W) * depth (D) * channels (C)

- $F_{tr}(\cdot, \theta)$**:** a transformation operation performed on a feature map **(x)** of size height **(h)** * width **(w)** * channels **(c1)** to obtain a new feature map of size height **(h)** * width **(w)** * channels (c2)

- $F_{sq}(\cdot)$**:** a **squeeze** operation

- $F_{ex}(\cdot, w)$**:** an **excite** operation

- $F_{scale}(\cdot)$**:** operation to multiply the feature importance (**weights**) with the original feature map (**x**)

A Squeeze-Excitation Residual Network (SEResNet50) [6] has been used for the present embodiment, as the primary architecture for modeling the regression problem.

SEResNets are a type of CNN that are better at learning important features from images than regular CNNs. They do this by explicitly modeling the relationships between the different channels in the feature maps. The Squeeze-Excite (SE) block in SEResNets has two components: a squeeze operation and an excitation operation.

The squeeze operation takes the feature maps from a previous layer and reduces them to a single-channel representation. This representation captures the overall importance of each channel. This is achieved by first squeezing the input feature maps along the spatial dimensions to obtain a channel descriptor for each channel. This channel descriptor captures the global importance of each channel. The squeeze operation is implemented using global average pooling, which simply averages the values in each channel across the spatial dimensions.

The excitation operation then takes this single channel representation and learns to adaptively recalibrate the channel-wise feature responses. This means that it learns to pay more attention to some channels and less attention to others, depending on the image. The excitation operation uses two fully connected (FC) layers, with a ReLU activation function in between. The first FC layer reduces the dimensionality of the channel descriptor, and the second FC layer produces the channel weights. The size of the feature channel is maintained through the SE operators because the output of the excitation operation is a set of weights that are multiplied by the input feature maps. This means that the number of channels in the input and output feature maps is the same.

In a typical CNN, each feature map has an equal weight whereas in SEResNet highly relevant feature maps have higher weights. Whereas the squeeze-and-excitation (SE) block can associate and learn the features between channels to improve the quality of feature representations using a feature recalibration strategy. This makes them better at learning from images and better at downstream tasks.

The used 3D-SEResNet50 network utilizes SE layers consisting of a stack of SE blocks as the encoder that is then connected to a FC layer. As commonly used in 3D image problems, 3D convolution layers operate on 3D volumetric data. The input to the network is a 3D CT volume of size $B \times C \times D \times H \times W$ where B is the batch size, C is the number of input channels, D is the depth, H is the height and W is the width of the 3D volume and the outputs are two values corresponding to the slope S and the intercept I of the calibration equation.

*Regression:*

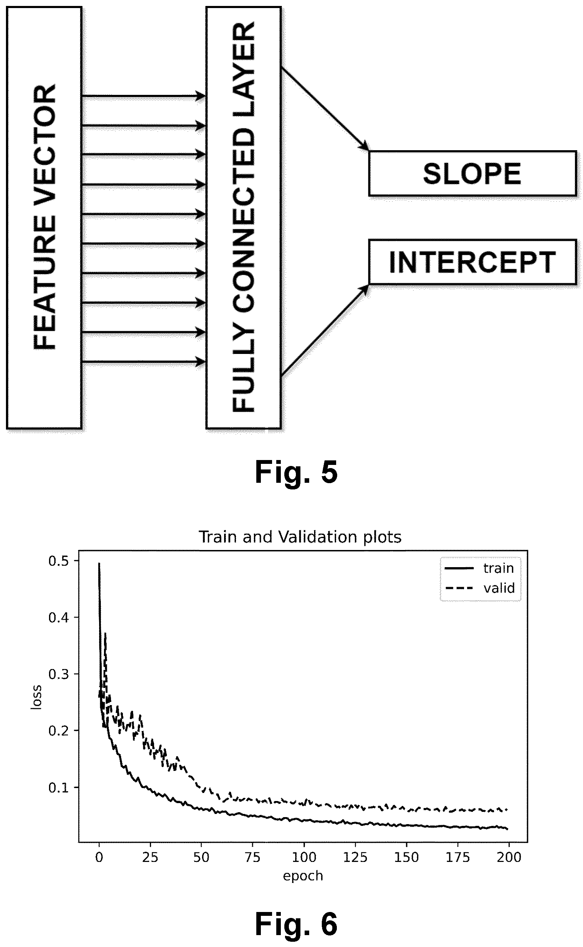

The linear regression module (i.e., the one identified in Figure 4 as "FC Layer") is composed of a single fully connected layer and takes the output feature vector of the CNN as the input. The output of this regression module is a vector of size 2 that corresponds to the slope and intercept of the calibration equation. This linear regression module is schematically shown in Figure 5.

*Loss functions:*

One of the most common loss functions for regression tasks is the mean squared error (MSE) and the same is here used to optimize the slope and intercept predictions. *x* is the predicted slope and intercept, and *y* refers to the ground truth slope and intercept.

The MSE loss is defined as follows:

$$\mathbf{L}_{MSE} = MSE(x, y) \qquad (1)$$

In order to regularize the network, the mean absolute error (MAE) is computed between the input volume calibrated with the predicted values *(V_x)* and the input volume calibrated with the ground truth values *(V_y)*. The L1 loss is given by:

$$\mathbf{L}_{l1} = MAE(V_x, V_y) \qquad (2)$$

The total loss function is defined as the summation of $L_{MSE}$ and $L_{L1}$ follows:

$$\mathbf{L}_{total} = MSE(x, y) + MAE(V_x, V_y) \qquad (3)$$

The network architecture was implemented in Python 3.7.0 using PyTorch framework 1.7.0 [4] and was trained on a single Nvidia RTX 3060 12 GB GPUs. The present inventors chose Evolved Sign Momentum (LiON) [5] as the optimizer with a learning rate of $1e^{-5}$ and a batch size of 4. No learning rate decay was used and the model was trained for 200 epochs for optimization.

*- Evaluation of the AI-based calibration model:*

The accuracy of the AI-based Phantomless calibration method of the first aspect of the present invention was evaluated against the ground truth phantom-based calibration method.

The CT scan database was divided into training, validation, and test sets with a split of 76%, 8%, and 16%, respectively. The test set was completely independent and was not used during the CNN training experiments. The ratio of data from different CT devices was maintained in the training, validation, and test sets. The CT scans were resized to 256x256x64. The performance of the CNN was assessed by comparing: (1) the calibration equations calculated using the CNN mathematical estimator and the ground truth phantom-based calibration equations, and (2) the BMD measured using the phantomless and the phantom-based calibrated images.

The following metrics were used: mean absolute error (MAE), the root mean squared error (RMSE), the Pearson's correlation coefficient (R) and the mean and standard deviation of the differences and percentage differences.

*Calibration laws evaluation:*

The CNN model was trained by minimizing the errors between the predicted values for the slope (a) and intercept (b) and the ones computed with the phantom-based calibration method. AI predicted phantomless calibrated laws were compared with the phantom-based calibration equations to validate the accuracy of the method.

*Bone mineral density measurements evaluation:*

Phantom-based and phantomless calibration laws were applied to the CT images of the test dataset to convert CT numbers to bone density values. Volumetric Bone Mineral Density (vBMD) was measured on both the phantom-based and phantomless calibrated images, and the results were compared.

vBMD was calculated at the proximal femur segmented in the CT image using semi-automated segmentation methods [7]. The cortical bone was segmented using methods developed in [8] and regions of interest corresponding to the cortical and trabecular bone were defined. Mean vBMD was calculated at the proximal femur region of interest in the trabecular, cortical and integral (trabecular + cortical) compartments.

The performance of the CNN phantomless approach, i.e., the method of the first aspect of the present invention, was compared to an alternative phantomless approach using a "generic" calibration law. The generic law was computed as the mean linear equation of the phantom-based calibration equations of the training dataset. The generic law *Density = a x HU + b* uses constant a & b coefficients for all the CT images of the test dataset. It will be used as benchmark against which the performance of the AI-based phantomless method will be evaluated.

- Results:

*AI-model performance:*

Obtaining an optimal fit is the goal of any deep-learning model. It is observed that the AI model/algorithm of the method of the first aspect of the present invention generalizes well due to the small gap between the train and the validation curves, as shown in Figure 6. As the curves of both training and validation decrease to a point of stability at 0.05, this indicates a well-fit model. As training is continued beyond 200 epochs, it is observed that there is no change in the plots and therefore, the training is stopped at 200 epochs.

*Calibration laws evaluation:*

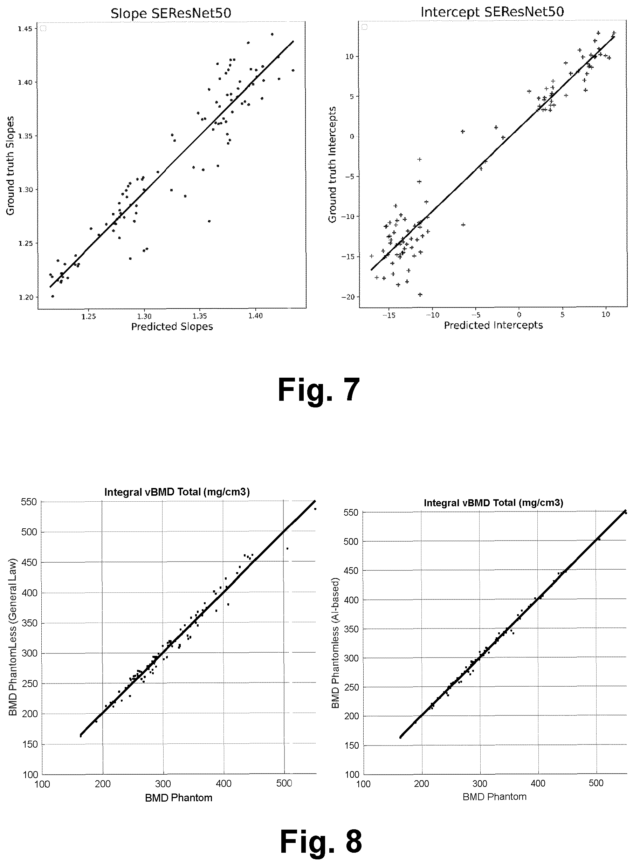

Figure 7 (left) shows the performance of the Phantomless calibration method of the first aspect of the present invention on the prediction of slope estimates when given a CT volume as input for the test dataset. A Pearson's correlation coefficient of 0.9453 between the predicted and ground truth slope values of the calibration law was achieved. The MAE on the slope values was 0.016 (mg/cm$^3$)/HU while the RMSE was 0.023 (mg/cm$^3$)/HU. Figure 7 (right) shows that the Phantomless calibration method of the first aspect of the present invention can accurately predict the intercept of the calibration law for CT volumes, with a Pearson's correlation of 0.9714 between the predicted and ground truth values. The MAE on the intercept values was 1.918 mg/cm$^3$ while the RMSE was 2.582 mg/cm$^3$. A high correlation between the predicted and ground truth values while simultaneously having low MAE and RMSE indicates a good performance by the AI model.

*Bone mineral density measurements evaluation:*

BMD measurements computed using phantom and phantomless calibration CT images were compared in order to evaluate the accuracy of the method.

The correlation coefficient R between the integral vBMD measured using the CT images calibrated with the generic law and the integral vBMD measured using the phantom-based calibrated CT images was 0.988, Figure 8 (left). A higher correlation was found between the integral vBMD measured using the AI-based calibrated CT images and the integral vBMD measured using the phantom-based calibrated CT images (R = 0.998), Figure 8 (right). The MAE and RMSE were lower when using the AI-based phantomless method, compared to the generic law, as shown in Table I. The random errors were found to be markedly decreased when using the AI-based phantomless method, compared to the generic law, with standard deviation (Std) of the percentage differences of 1.448%, against 3.214%.

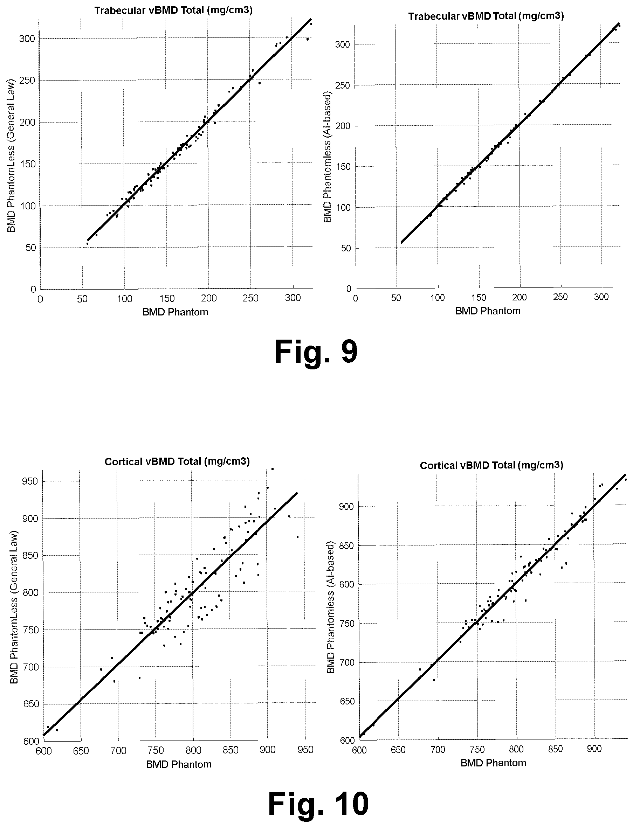

The correlation coefficient R between the trabecular vBMD measured using the CT images calibrated with the generic law and the trabecular vBMD measured using the phantom-based calibrated CT images was 0.994, Figure 9 (left). A slightly higher correlation was found between the trabecular vBMD measured using the AI-based calibrated CT images and the trabecular vBMD measured using the phantom-based calibrated CT images (R = 0.999), Figure 9 (right). The MAE and RMSE were lower when using the AI-based phantomless method, compared to the generic law, as shown in Table I. The random errors were found to be markedly decreased when using the AI-based phantomless method, compared to the generic law, with standard deviation (Std) of the percentage differences of 1.545%, against 4.049%.

The correlation coefficient R between the cortical vBMD measured using the CT images calibrated with the generic law and the cortical vBMD measured using the phantom-based calibrated CT images was 0.9106, Figure 10 (left). The improvement in correlation with the AI-based phantomless method of the present invention is more notable for high BMD values, such as the cortical vBMD. The correlation between the cortical vBMD measured using the CT images calibrated with the AI-based method and the cortical vBMD measured using the phantom-based calibrated CT images was found to be much higher (R = 0.9835, Figure 10 (right)). The MAE and RMSE were also found to be lower for the AI-based phantomless method, compared to the generic law, as shown in Table I. The random errors were found to be markedly decreased when using the AI-based phantomless method, compared to the generic law, with standard deviation (Std) of the percentage differences of 1.467%, against 3.425%.

*Table I: Accuracy of vBMD measurements (in mg/cm$^3$) using the general law or the AI-based approach against the phantom-based approach.*

| | | R | MAE | RMSE | Difference | | Difference (%) | |
|---|---|---|---|---|---|---|---|---|
| | | | | | Mean | Std | Mean | Std |
| Integral vBMD | General Law | 0.988 | 8.493 | 10.764 | 0.033 | 10.818 | 0.054 | 3.214 |
| | AI-based | 0.998 | 3.268 | 4.383 | 0.282 | 4.396 | 0.102 | 1.448 |
| Trabecular vBMD | General Law | 0.994 | 4.948 | 6.117 | 0.511 | 6.127 | 0.558 | 4.049 |
| | AI-based | 0.999 | 1.757 | 2.348 | 0.308 | 2.339 | 0.227 | 1.545 |
| Cortical vBMD | General Law | 0.911 | 22.560 | 28.354 | -1.962 | 28.429 | -0.211 | 3.425 |
| | AI-based | 0.983 | 8.715 | 11.792 | 0.104 | 11.851 | 0.026 | 1.467 |

The performance of the method of the first aspect of the invention can also be evaluated against state-of-art phantomless calibration methods.

Particularly, Boden et al. [9] reported an R value of 0.96 for trabecular bone at the vertebral body using two CT devices from the same manufacturer at the same institution from 48 healthy volunteers. Weaver et al. [10] reported an R$^2$ value of 0.87 for trabecular bone at the vertebral body. Lee et al. [11] reported R value of 0.998 for the lumbar spine and an R value of 0.984 for the hip using 40 scans from each region acquired on nine different CT scanner models from 24 different scanners. Eggermont et al. [12] evaluated the failure load of bone metastases from 57 patients using air-fat-muscle (AFM) calibration and reported an R$^2$ value of 0.94. Prado et al. [3] investigated the opportunistic application of phantom-less calibration methods for fracture risk prediction using QCT/FEA and reported an R$^2$ value of 0.99 for trabecular bone at L3.

As evidenced by the above results of the experimental embodiment developed in the present section, the method of the first aspect of the present invention implements an AI-based model to calibrate Phantomless CT images. AI-based phantomless calibration of CT images performed better than ground truth average calibration law. AI-derived BMD measurements were also more accurate than estimates obtained using a generic calibration law, especially for high BMD values. These results indicate that AI techniques can achieve fully automatic phantomless calibration of CT images.

There are several exciting avenues of future work that lie ahead. In the developed experimental embodiment, 3D CT volume has been used as input to the model. However, it is also possible to use 2D slice/slices, 2.5D volumes as input. The AI model has been trained from scratch, so that the network weights have been initialized far from the global optima. An interesting approach here would be the use of transfer learning, which would be useful to train the model for other body parts. A deep learning model trained on CT images of the femur, independent of the problem statement, can be used as a starting point. This would enable the AI model to initialize away from the local optima and closer to the global optima. This enables the already trained model to easily tune to the new optimization task.

A person skilled in the art could introduce changes and modifications in the embodiments described without departing from the scope of the invention as it is defined in the attached claims.

References:

[1] C. Winsor, X. Li, M. Qasim, C.R. Henak, P.J. Pickhardt, H. Ploeg, M. Viceconti, Evaluation of patient tissue selection methods for deriving equivalent density calibration for femoral bone quantitative CT analyses, Bone. 143 (2021). doi:10.1016/j.bone.2020.115759.

[2] Bryn Matheson, N. Neeteson, S.K. Boyd, Establishing error bounds for internal calibration on quantitative computed tomography (QCT), in: ASBMR, 2023.

[3] M. Prado, S. Khosla, C. Chaput, H. Giambini, Opportunistic application of phantom-less calibration methods for fracture risk prediction using QCT/FEA, Eur. Radiol. 31 (2021) 9428-9435. doi:10.1007/s00330-021-08071-w.

[4] A. Paszke, S. Gross, F. Massa, A. Lerer, J. Bradbury, G. Chanan, T. Killeen, Z. Lin, N. Gimelshein, L. Antiga, A. Desmaison, Pytorch: An imperative style, high-performance deep learning library., in: Adv. Neural Inf. Process. Syst., 2019.

[5] X. Chen, C. Liang, D. Huang, E. Real, K. Wang, Y. Liu, H. Pham, X. Dong, T. Luong, C.J. Hsieh, Y. Lu, Symbolic discovery of optimization algorithms, ArXiv Prepr. ArXiv2302.06675. (2023).

[6] J. Hu, L. Shen, G. Sun, Squeeze-and-excitation networks, in: Proc. IEEE Conf. Comput. Vis. Pattern Recognit., 2018: pp. 7132-7141.

[7] L. Humbert, Y. Martelli, R. Fonollà, M. Steghöfer, S. Di Gregorio, L.M.L.M. del Río Barquero, 3D-DXA: Analyzing the Femoral Shape, the Trabecular Macrostructure and the Cortical layer in 3D from DXA images, IEEE Trans. Med. Imaging. 36 (2017) 27-39. doi:10.1109/TMI.2016.2593346.

[8] L. Humbert, J. Hazrati Marangalou, L.M. Del Río Barquero, G.H. van Lenthe, B. van Rietbergen, Technical Note: Cortical thickness and density estimation from clinical CT using a prior thickness-density relationship, Med. Phys. 43 (2016) 1945-1954. doi:10.1118/1.4944501.

[9] S.D. Boden, D.J. Goodenough, C.D. Stockham, E. Jacobs, T. Dina, R.M. Allman, Precise measurement of vertebral bone density using computed tomography without the use of an external reference phantom, J. Digit. Imaging. 2 (1989) 31-38. doi:10.1007/BF03168013.

[10] A.A. Weaver, Kristen M. Beavers, R.C. Hightower, S.K. Lynch, A.N. Miller, J.D. Stitzel, Lumbar Bone Mineral Density Phantom-less Computed Tomography Measurements and Correlation with Age and Fracture Incidence, Traffic Inj Prev. 16 (2015). doi:10.1080/15389588.2015.1054029.Lumbar.

[11] Y.H. Lee, J.J. Kim, I.G. Jang, Patient-Specific Phantomless Estimation of Bone Mineral Density and Its Effects on Finite Element Analysis Results: A Feasibility Study, Comput. Math. Methods Med. 2019 (2019). doi:10.1155/2019/4102410.

[12] F. Eggermont, N. Verdonschot, Y. van der Linden, E. Tanck, Calibration with or without phantom for fracture risk prediction in cancer patients with femoral bone metastases using CT-based finite element models, PLoS One. 14 (2019).

**Claims**

1. A computer-implemented method for phantomless calibration of medical images, comprising:

    - obtaining medical images without calibration phantoms of a plurality of previous subjects;
    - obtaining calibration laws for said obtained medical images without calibration phantoms;
    - training an artificial intelligence algorithm with at least one dataset of said obtained medical images without calibration phantoms and training said artificial intelligence algorithm also with said calibration laws, to learn a mathematical estimator that infers from the image data the calibration coefficients of the calibration laws; and
    - calibrating with said at least one learned mathematical estimator at least one medical image of a current subject acquired without using a calibration phantom;

    wherein the method further comprises:

    - receiving and/or accessing medical images of said plurality of previous subjects scanned together with calibration phantoms; and
    - computing said calibration laws from said received and/or accessed medical images;

    and wherein said step of obtaining medical images without calibration phantoms comprises:

    - generating medical images without calibration phantoms by removing, cropping or hiding image portions representing calibration phantoms from medical images of said plurality of previous subjects scanned together with calibration phantoms; and/or

- receiving and/or accessing medical images of said plurality of previous subjects scanned without calibration phantoms.

2. The computer-implemented method of claim 1, wherein the medical images of the plurality of previous subjects scanned with calibration phantoms and the medical images of the plurality of previous subjects obtained without calibration phantoms are from or include the same body part of a plurality of previous subjects.

3. The computer-implemented method of any of the previous claims, further comprising performing quantitative measurements, on one or more regions of interests, of at least one of the following parameters of interest on said at least one calibrated medical image of a current subject acquired without using a calibration phantom: bone mineral density, mineral content in blood vessels, hepatic-fat content in the liver, and grey-to-white matter ratio in the brain, or a combination thereof.

4. The computer-implemented method of any of the previous claims, wherein said calibration laws are linear equations.

5. The computer-implemented method of any of the previous claims, wherein said obtained medical images without calibration phantoms and said at least one medical image of a current subject acquired without using a calibration phantom are computed tomography images and/or magnetic resonance images.

6. The computer-implemented method of any of the previous claims, wherein said at least one dataset comprises a training dataset and a validation dataset, said training step comprising inputting the same with said training dataset and said validation dataset and with said calibration laws, and wherein the method further comprises testing said trained artificial intelligence algorithm by inputting the same with a test dataset of said obtained medical images without calibration phantoms.

7. The computer-implemented method of any of the previous claims, comprising using said artificial intelligence algorithm to obtain said mathematical estimator by processing the whole area or volume of said obtained medical images without calibration phantoms, or of preprocessed images obtained therefrom, without segmenting or selecting specific image areas.

8. The computer-implemented method of any of the previous claims, wherein said obtained medical images without calibration phantoms of the plurality of previous subjects and said at least one medical image of a current subject acquired without using a calibration phantom represent or include a representation of the same body part.

9. The computer-implemented method of any of claims 1 to 8, wherein said received and/or accessed medical images of the plurality of previous subjects scanned without a calibration phantom were scanned with the same scanning system as said received and/or accessed medical images of the plurality of previous subjects scanned together with a calibration phantom.

10. The computer-implemented method of any of the previous claims, wherein said artificial intelligence algorithm is a deep learning algorithm.

11. The computer-implemented method of claim 10, wherein said deep learning algorithm implements a convolutional neural network or a neural network of compact convolutional transformers.

12. A system for phantomless calibration of medical images, comprising a processing device including a processor and a computer-readable medium having encoded thereon computer-executable instructions to cause the processor to execute the computer-implemented method according to any of claims 1 to 11.

13. A computer program, including code instructions that when executed on at least one processor perform the steps of the method of any of claims 1 to 11, for phantomless calibration of medical images.

**Patentansprüche**

1. Computerimplementiertes Verfahren zur phantomfreien Kalibrierung medizinischer Bilder, umfassend:

- Erhalten von medizinischen Bildern ohne Kalibrierungsphantome von mehreren vorhergehenden Probanden;

- Erhalten von Kalibrierungsgesetzen für die erhaltenen medizinischen Bilder ohne Kalibrierungsphantome;
- Trainieren eines Algorithmus künstlicher Intelligenz mit mindestens einem Datensatz der erhaltenen medizinischen Bilder ohne Kalibrierungsphantome und Trainieren des Algorithmus künstlicher Intelligenz außerdem mit den Kalibrierungsgesetzen, um einen mathematischen Schätzer zu erlernen, der aus den Bilddaten die Kalibrierungskoeffizienten der Kalibrierungsgesetze inferiert; und
- Kalibrieren mindestens eines medizinischen Bildes eines aktuellen Probanden, das ohne Verwendung eines Kalibrierungsphantoms aufgenommen wurde, mit dem mindestens einen erlernten mathematischen Schätzer;

wobei das Verfahren ferner umfasst:

- Empfangen von medizinischen Bildern und/oder Zugreifen auf medizinische Bilder der mehreren vorhergehenden Probanden, die zusammen mit Kalibrierungsphantomen gescannt wurden; und
- Berechnen der Kalibrierungsgesetze aus den empfangenen medizinischen Bildern und/oder medizinischen Bildern, auf die zugegriffen wurde;

wobei der Schritt des Erhaltens von medizinischen Bildern ohne Kalibrierungsphantome umfasst:

- Erzeugen medizinischer Bilder ohne Kalibrierungsphantome durch Entfernen, Beschneiden oder Verbergen von Bildabschnitten, die Kalibrierungsphantome darstellen, aus medizinischen Bildern der mehreren vorhergehenden Probanden, die zusammen mit Kalibrierungsphantomen gescannt wurden; und/oder
- Empfangen von medizinischen Bildern und/oder Zugreifen auf medizinische Bilder der mehreren vorhergehenden Probanden, die ohne Kalibrierungsphantome gescannt wurden.

2. Computerimplementiertes Verfahren nach Anspruch 1, wobei die medizinischen Bilder der mehreren vorhergehenden Probanden, die mit Kalibrierungsphantomen gescannt wurden, und die medizinischen Bilder der mehreren vorhergehenden Probanden, die ohne Kalibrierungsphantome erhalten wurden, von demselben Körperteil mehrerer vorhergehender Probanden stammen oder diesen enthalten.

3. Computerimplementiertes Verfahren nach einem der vorhergehenden Ansprüche, ferner umfassend Durchführen quantitativer Messungen an einem oder mehreren Bereichen von Interesse von mindestens einem der folgenden Parameter von Interesse auf dem mindestens einen kalibrierten medizinischen Bild eines aktuellen Probanden, das ohne Verwendung eines Kalibrierungsphantoms aufgenommen wurde: Knochenmineraldichte, Mineralgehalt in Blutgefäßen, Leberfettgehalt in der Leber und Grau-Weiß-Verhältnis im Gehirn oder eine Kombination davon.

4. Computerimplementiertes Verfahren nach einem der vorhergehenden Ansprüche, wobei die Kalibrierungsgesetze lineare Gleichungen sind.

5. Computerimplementiertes Verfahren nach einem der vorhergehenden Ansprüche, wobei die erhaltenen medizinischen Bilder ohne Kalibrierungsphantome und das mindestens eine medizinische Bild eines aktuellen Probanden, das ohne Verwendung eines Kalibrierungsphantoms aufgenommen wurde, Computertomografiebilder und/oder Magnetresonanzbilder sind.

6. Computerimplementiertes Verfahren nach einem der vorhergehenden Ansprüche, wobei der mindestens eine Datensatz einen Trainingsdatensatz und einen Validierungsdatensatz umfasst, wobei der Trainingsschritt Eingeben desselben mit dem Trainingsdatensatz und dem Validierungsdatensatz und mit den Kalibrierungsgesetzen umfasst und wobei das Verfahren ferner Prüfen des trainierten Algorithmus künstlicher Intelligenz durch Eingeben desselben mit einem Prüfdatensatz der erhaltenen medizinischen Bilder ohne Kalibrierungsphantome umfasst.

7. Computerimplementiertes Verfahren nach einem der vorhergehenden Ansprüche, umfassend Verwenden des Algorithmus künstlicher Intelligenz, um den mathematischen Schätzer zu erhalten, durch Verarbeiten des gesamten Bereichs oder Volumens der erhaltenen medizinischen Bilder ohne Kalibrierungsphantome oder von vorverarbeiteten Bildern, die davon erhalten wurden, ohne Segmentieren oder Auswählen spezifischer Bildbereiche.

8. Computerimplementiertes Verfahren nach einem der vorhergehenden Ansprüche, wobei die erhaltenen medizinischen Bilder ohne Kalibrierungsphantome der mehreren vorhergehenden Probanden und das mindestens eine medizinische Bild eines aktuellen Probanden, das ohne Verwendung eines Kalibrierungsphantoms aufgenommen wurde, eine Darstellung des gleichen Körperteils darstellen oder enthalten.

9. Computerimplementiertes Verfahren nach einem der Ansprüche 1 bis 8, wobei die empfangenen medizinischen Bilder der mehreren vorhergehenden Probanden und/oder der medizinischen Bilder, auf die zugegriffen wurde, die ohne ein Kalibrierungsphantom gescannt wurden, mit demselben Scansystem gescannt wurden wie die empfangenen und/oder zugegriffenen medizinischen Bilder der mehreren vorhergehenden Probanden, die zusammen mit einem Kalibrierungsphantom gescannt wurden.

10. Computerimplementiertes Verfahren nach einem der vorhergehenden Ansprüche, wobei der Algorithmus künstlicher Intelligenz ein "Deep Learning"-Algorithmus ist.

11. Computerimplementiertes Verfahren nach Anspruch 10, wobei der "Deep Learning"-Algorithmus ein neuronales Faltungsnetz oder ein neuronales Netz kompakter Faltungstransformatoren implementiert.

12. System zur phantomfreien Kalibrierung medizinischer Bilder, umfassend eine Verarbeitungsvorrichtung einschließlich eines Prozessors und eines computerlesbaren Mediums, auf dem computerausführbare Anweisungen codiert sind, um zu bewirken, dass der Prozessor das computerimplementierte Verfahren nach einem der Ansprüche 1 bis 11 ausführt.

13. Computerprogramm, das Codeanweisungen enthält, die dann, wenn sie auf mindestens einem Prozessor ausgeführt werden, die Schritte des Verfahrens nach einem der Ansprüche 1 bis 11 zur phantomfreien Kalibrierung medizinischer Bilder durchführen.


**Revendications**

1. Procédé mis en œuvre par ordinateur pour étalonnage sans fantôme d'images médicales, comprenant :

   - l'obtention d'images médicales sans fantômes d'étalonnage d'une pluralité de sujets précédents ;
   - l'obtention de lois d'étalonnage pour lesdites images médicales obtenues, sans fantômes d'étalonnage ;
   - l'entraînement d'un algorithme d'intelligence artificielle avec au moins un ensemble de données desdites images médicales obtenues sans fantômes d'étalonnage et l'entraînement dudit algorithme d'intelligence artificielle également avec lesdites lois d'étalonnage, à l'apprentissage d'un estimateur mathématique qui déduit des données d'image les coefficients d'étalonnage des lois d'étalonnage ; et
   - l'étalonnage avec ledit au moins un estimateur mathématique appris d'au moins une image médicale d'un sujet actuel acquise sans utiliser de fantôme d'étalonnage ;

   dans lequel le procédé comprend en outre :

   - la réception de et/ou l'accès à des images médicales de ladite pluralité de sujets précédents scannés avec des fantômes d'étalonnage ; et
   - le calcul desdites lois d'étalonnage à partir des images médicales reçues et/ou accédées ;

   et dans lequel ladite étape d'obtention d'images médicales sans fantômes d'étalonnage comprend :

   - la génération d'images médicales sans fantômes d'étalonnage en supprimant, en recadrant ou en cachant des zones d'image représentant des fantômes d'étalonnage à partir d'images médicales de ladite pluralité de sujets précédents scannés avec des fantômes d'étalonnage ; et/ou
   - la réception de et/ou l'accès à des images médicales de ladite pluralité de sujets précédents scannés sans fantômes d'étalonnage.

2. Procédé mis en œuvre par ordinateur selon la revendication 1, dans lequel les images médicales de la pluralité de sujets précédents scannés avec des fantômes d'étalonnage et les images médicales de la pluralité de sujets précédents obtenues sans fantômes d'étalonnage proviennent de ou comprennent la même partie du corps d'une pluralité de sujets précédents.

3. Procédé mis en œuvre par ordinateur selon l'une quelconque des revendications précédentes, comprenant en outre la réalisation de mesures quantitatives, sur une ou plusieurs régions d'intérêt, d'au moins un des paramètres d'intérêt suivants sur ladite au moins une image médicale étalonnée d'un sujet actuel acquise sans utilisation d'un fantôme d'étalonnage : densité minérale osseuse, teneur en minéraux dans les vaisseaux sanguins, teneur en graisse hépatique dans le foie, et rapport matière grise/matière blanche dans le cerveau, ou une combinaison de ceux-ci.

4. Procédé mis en œuvre par ordinateur selon l'une quelconque des revendications précédentes, dans lequel lesdites lois d'étalonnage sont des équations linéaires.

5. Procédé mis en œuvre par ordinateur selon l'une quelconque des revendications précédentes, dans lequel lesdites images médicales obtenues sans fantômes d'étalonnage et ladite au moins une image médicale d'un sujet actuel acquise sans utiliser de fantôme d'étalonnage sont des images de scanner et/ou des images de résonance magnétique.

6. Procédé mis en œuvre par ordinateur selon l'une quelconque des revendications précédentes, dans lequel ledit au moins un ensemble de données comprend un ensemble de données d'entraînement et un ensemble de données de validation, ladite étape d'entraînement comprenant la saisie dans celle-ci dudit ensemble de données d'entraînement et dudit ensemble de données de validation, ainsi que desdites lois d'étalonnage, et dans lequel le procédé comprend en outre le test dudit algorithme d'intelligence artificielle entraîné par la saisie dans celui-ci d'un ensemble de données de test desdites images médicales obtenues sans fantômes d'étalonnage.

7. Procédé mis en œuvre par ordinateur selon l'une quelconque des revendications précédentes, comprenant l'utilisation dudit algorithme d'intelligence artificielle pour obtenir ledit estimateur mathématique en traitant l'ensemble de la zone ou du volume desdites images médicales obtenues sans fantômes d'étalonnage, ou d'images prétraitées obtenues à partir de celles-ci, sans segmenter ou sélectionner des zones spécifiques de l'image.

8. Procédé mis en œuvre par ordinateur selon l'une quelconque des revendications précédentes, dans lequel lesdites images médicales obtenues sans fantômes d'étalonnage de la pluralité de sujets précédents et ladite au moins une image médicale d'un sujet actuel acquise sans utiliser de fantôme d'étalonnage représentent ou comprennent une représentation de la même partie du corps.

9. Procédé mis en œuvre par ordinateur selon l'une quelconque des revendications 1 à 8, dans lequel les images médicales reçues et/ou consultées de la pluralité de sujets précédents scannés sans fantôme d'étalonnage ont été scannées avec le même système de balayage que lesdites images médicales reçues et/ou consultées de la pluralité de sujets précédents scannées avec un fantôme d'étalonnage.

10. Procédé mis en œuvre par ordinateur selon l'une quelconque des revendications précédentes, dans lequel ledit algorithme d'intelligence artificielle est un algorithme d'apprentissage profond.

11. Procédé mis en œuvre par ordinateur selon la revendication 10, dans lequel ledit algorithme d'apprentissage profond met en œuvre un réseau neuronal convolutif ou un réseau neuronal de transformateurs convolutifs compacts.

12. Système d'étalonnage sans fantôme d'images médicales, comprenant un dispositif de traitement incluant un processeur et un support lisible par ordinateur sur lequel sont encodées des instructions exécutables par ordinateur pour amener le processeur à exécuter le procédé mis en œuvre par ordinateur selon l'une quelconque des revendications 1 à 11.

13. Programme d'ordinateur, comprenant des instructions de code qui, lorsqu'elles sont exécutées sur au moins un processeur, réalisent les étapes du procédé selon l'une quelconque des revendications 1 à 11, pour l'étalonnage sans fantôme d'images médicales.

# Fig. 1A

# Fig. 1B

Original CT <u>with</u> phantom

Simulated CT <u>without</u> phantom

# Fig. 2

Input volume

Data Preprocessing

Mathematical Estimator

Calibration

Calibrated volume

# Fig. 3

Input 3D Volume
H x W x D x C

$x$

$h$

$w$

$c_1$

$F_{tr}(., \theta)$

$h$

$w$

$c_2$

$F_{sq}(.)$

$F_{ex}(., w)$

$F_{scale}(.)$

$\tilde{x}$

$h$

$w$

$c_2$

FC Layer

S

I

### Squeeze

- Shrinking feature maps through spatial dimension
- Global distribution of channel-wise responses

### Excitation

- Learning to explicitly model channel association
- Gating mechanism to produce channel-wise weights

### Scale

- Reweighting the feature maps

Multiple SE blocks

**Fig. 4**

Fig. 5

Fig. 6

**Fig. 7**

**Fig. 8**

## Fig. 9

## Fig. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101750108 B1

- US 2020357150 A1

**Non-patent literature cited in the description**

- **C. WINSOR ; X. LI ; M. QASIM ; C.R. HENAK ; P.J. PICKHARDT ; H. PLOEG ; M. VICECONTI**. Evaluation of patient tissue selection methods for deriving equivalent density calibration for femoral bone quantitative CT analyses. *Bone*, 2021, vol. 143
- **BRYN MATHESON ; N. NEETESON ; S.K. BOYD**. Establishing error bounds for internal calibration on quantitative computed tomography (QCT). *ASBMR*, 2023
- **M. PRADO ; S. KHOSLA ; C. CHAPUT ; H. GIAMBINI**. Opportunistic application of phantomless calibration methods for fracture risk prediction using QCT/FEA. *Eur. Radiol.*, 2021, vol. 31, 9428-9435
- **A. PASZKE ; S. GROSS ; F. MASSA ; A. LERER ; J. BRADBURY ; G. CHANAN ; T. KILLEEN ; Z. LIN ; N. GIMELSHEIN ; L. ANTIGA**. Pytorch: An imperative style, high-performance deep learning library. *Adv. Neural Inf. Process. Syst*, 2019
- **X. CHEN ; C. LIANG ; D. HUANG ; E. REAL ; K. WANG ; Y. LIU ; H. PHAM ; X. DONG ; T. LUONG ; C.J. HSIEH**. Symbolic discovery of optimization algorithms. *ArXiv Prepr. ArXiv2302.06675*, 2023
- **J. HU ; L. SHEN ; G. SUN**. Squeeze-and-excitation networks. *Proc. IEEE Conf. Comput. Vis. Pattern Recognit.*, 2018, 7132-7141
- **L. HUMBERT ; Y. MARTELLI ; R. FONOLLÀ ; M. STEGHÖFER ; S. DI GREGORIO ; L.M.L.M. DEL RÍO BARQUERO**. 3D-DXA: Analyzing the Femoral Shape, the Trabecular Macrostructure and the Cortical layer in 3D from DXA images. *IEEE Trans. Med. Imaging.*, 2017, vol. 36, 27-39

- **L. HUMBERT ; J. HAZRATI MARANGALOU ; L.M. DEL RÍO BARQUERO ; G.H. VAN LENTHE ; B. VAN RIETBERGEN**. Technical Note: Cortical thickness and density estimation from clinical CT using a prior thickness-density relationship. *Med. Phys.*, 2016, vol. 43, 1945-1954
- **S.D. BODEN ; D.J. GOODENOUGH ; C.D. STOCKHAM ; E. JACOBS ; T. DINA ; R.M. ALLMAN**. Precise measurement of vertebral bone density using computed tomography without the use of an external reference phantom. *J. Digit. Imaging.*, 1989, vol. 2, 31-38
- **A.A. WEAVER ; KRISTEN M. BEAVERS ; R.C. HIGHTOWER ; S.K. LYNCH ; A.N. MILLER ; J.D. STITZEL**. Lumbar Bone Mineral Density Phantomless Computed Tomography Measurements and Correlation with Age and Fracture Incidence. *Traffic Inj Prev*, 2015, vol. 16
- **Y.H. LEE ; J.J. KIM ; I.G. JANG**. Patient-Specific Phantomless Estimation of Bone Mineral Density and Its Effects on Finite Element Analysis Results: A Feasibility Study. *Comput. Math. Methods Med*, 2019, vol. 2019
- **F. EGGERMONT ; N. VERDONSCHOT ; Y. VAN DER LINDEN ; E. TANCK**. Calibration with or without phantom for fracture risk prediction in cancer patients with femoral bone metastases using CT-based finite element models. *PLoS One.*, 2019, vol. 14